(19) Europäisches Patentamt
European Patent Office
Office européen des brevets

(11) Publication number: **0 086 454**
**A1**

# (12) EUROPEAN PATENT APPLICATION

(21) Application number: **83101236.4**

(22) Date of filing: **09.02.83**

(51) Int. Cl.³: **C 07 D 209/48**

(30) Priority: **12.02.82 US 348652**

(43) Date of publication of application: **24.08.83**
**Bulletin 83/34**

(84) Designated Contracting States: **BE CH DE FR GB IT LI NL**

(71) Applicant: **J.T. Baker Chemical Co., 222 Red School Lane, Phillipsburg, New Jersey 08865 (US)**

(72) Inventor: **Gray, Gary M., 1572 Ralston Road, Behtlehem Pennsylvania (US)**

(74) Representative: **Vossius Vossius Tauchner Heunemann Rauh, Siebertstrasse 4 P.O. Box 86 07 67, D-8000 München 86 (DE)**

## (54) Preparation of trans-zeatin intermediate.

(57) An improved synthesis for (E)-2(4-hydroxy-3-methyl-2-butenyl)-1H-isoindole-1,3(2H)-dione, an intermediate for the production of trans-zeatin, utilizes (E)-4-halo-2-methylbut-2-en-1-al or an aldehyde protected derivative thereof and an alkali metal phthalimide as starting materials.

EP 0 086 454 A1

Our Ref.: S 241 EP
Case: B-1190 EP

VOSSIUS · VOSSIUS · TAUCHNER
HEUNEMANN · RAUH
PATENTANWÄLTE
SIEBERTSTR. 4, 8000 MÜNCHEN 80
TEL. (089) 47 40 75

9. Feb. 1983

J. T. Baker Chemical Company
Phillipsburg, New Jersey, U.S.A.

# PREPARATION OF TRANS-ZEATIN INTERMEDIATE

## Field of the Invention

This invention relates to a process for the production of an intermediate useful for the preparation of trans-zeatin.

## Background of the Invention

One of the most effective natural stimulants of plant cell division is trans-zeatin. trans-Zeatin is employed to induce cells to multiply and for cell enlargement and is a truly effective cytokinin plant growth regulator. However, the methods for synthesis of trans-zeatin and the intermediate therefor are undesirably long and complex, employ starting materials that are generally not readily available and result in relatively low yields of trans-zeatin that are not as isomerically pure as desirable. There is therefore a need for a synthetic method that would be shorter, employ more readily available starting materials and which would result in higher yields of isomerically pure products.

It is known that trans-zeatin can be produced from (E)-2(4-hydroxy-3-methyl-2-butenyl)-1H-isoindole-1,3(2H)-dione by removal of the phthalimide group by reaction of said compound with hydrazine and condensing the resulting product with 6-chloropurine to give trans-zeatin. Such a procedure is disclosed by Joseph Corse and Judith Kuhnle, "An Improved Synthesis of trans-Zeatin", Synthesis, pp. 618-619, November, 1972. However, the methods for the preparation of this intermediate suffer from the hereinbefore stated disadvantages. Thus, a new and improved method for the synthesis of this intermediate that eliminates or substantially reduces the disadvantages of the prior art synthetic methods is highly desirable.

## Summary of the Invention

An improved method for the production of (E)-2(4-hydroxy-3-methyl-2-butenyl)-1H-isoindole-1,3(2H)-dione comprises reacting (1) a (E)-4-halo-2-methylbut-2-en-1-al compound or a derivative thereof wherein the aldehyde moiety is blocked or protected with (2) an alkali metal phthalimide and removing any aldehyde protective groups from the reaction product

by hydrolysis thereof to form (E)-2(3-formyl-2-butenyl)-1H-isoindole-1,3(2H)-dione and reducing said compound with a suitable reducing agent to form (E)-2(4-hydroxy-3-methyl-2-butenyl)-1H-isoindole-1,3(2H)-dione.

## Detailed Description of the Invention

The improved method of this invention comprises reacting (E)-4-halo-2-methylbut-2-en-1-al or a blocked derivative thereof, preferably a compound of the formula

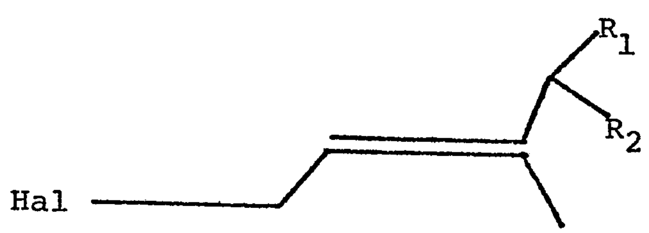

wherein Hal is chlorine or bromine, $R_1$ and $R_2$ are aldehyde protective groups, preferably lower alkoxy having from 1 to 4 carbon atoms in the alkoxy group or $R_1$ and $R_2$ taken together are =O, with an alkali metal phthalimide of the formula

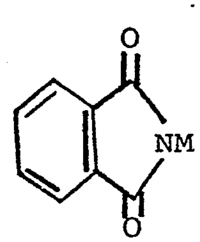

wherein M is any suitable alkali metal, preferably sodium or potassium. This addition reaction to form a compound of the formula

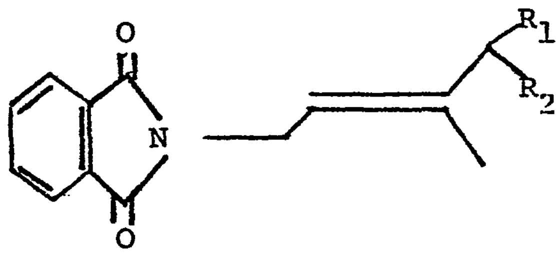

wherein $R_1$ and $R_2$ are as defined hereinbefore can be conducted at room temperature or with suitable heating if desired and is conducted in a suitable polar organic solvent such as for example, acetone, tetrahydrofuran or dimethylformamide, preferably dry dimethylformamide.

When one employs a (E)-4-halo-2-methylbut-2-en-1-al as a reactant (E)-2(3-formyl-2-butenyl)-1H-isoindole-1,3(2H)-dione is obtained directly whereas if an aldehyde blocked or protected derivative of (E)-4-halo-2-methylbut-2-en-1-al is employed as the reactant, the reaction product must be hydrolyzed to remove the protective group to obtain (E)-2(3-formyl-2-butenyl)-1H-isoindole-1,3(2H)-dione. Any suitable hydrolysis conditions may be employed, however, hydrolysis of the reaction product is preferably conducted in an acetone-water mixture with

any suitable weak acid catalyst, such as for example, p-toluene sulfonic acid, acetic acid, or dilute phosphoric or sulfuric acid.

When the (E)-2(3-formyl-2-butenyl)-1H-isoindole-1,3(2H)-dione is obtained, either directly or by hydrolysis, it is reduced to the desired (E)-2(4-hydroxy-3-methyl-2-butenyl)-1H-isoindole-1,3(2H)-dione by reduction thereof with any suitable reducing agent that will reduce the aldehyde moiety but not the imide moiety, such as sodium or lithium borohydride, aluminum hydride or an alkali metal cyanoborohydride. The reduction reaction is carried out in any suitable solvent such as an etheral solvent such as tetrahydrofuran, dioxane or ether and is preferably carried out with sodium cyanoborohydride in tetrahydrofuran. The reaction is preferably conducted at a pH of about 3 and at a low temperature of about -10 to about 20°C.

The improved process of this invention is illustrated by but is not limited to the following example.

EXAMPLE

A mixture of 5.95g (25.0 mmol) of crude (E)-4-bromo-1,1-diethoxy-2-methyl-2-butene and 4.63g (25.0 mmol) of potassium phthalimide in 40 ml of dry N,N-dimethylformamide was stirred under nitrogen at room temperature for about two hours and then

the mixture poured into 250 ml deionized water. The insoluble residue was extracted with two 150 ml portions of methylene chloride which were combined and washed with five 200 ml portions of deionized water before being evaporated to dryness.

The yellow oily residue was taken up in 50 ml of acetone and 10 ml deionized water and this solution stirred for about 30 minutes after 1.0g p-toluene sulfonic acid monohydrate had been added. The solution was evaporated to dryness.

The yellow solid residue was dissolved in a mixture of 100 ml of 10% potassium carbonate solution and 100 ml methylene chloride. The phases were separated and the aqueous layer washed with an additional 50 ml methylene chloride which was then combined with the organic phase. The methylene chloride solution was washed with four 100 ml portions of water before being treated with anhydrous magnesium sulfate and a charcoal decolorizer. Following gravity filtration the solution was evaporated to dryness to yield 4.80g of pale yellow solid crude product for a 83.5% yield. The crude product was recrystallized from a mixture of methylene chloride and hexane to yield 2.36g (E)-2(3-formyl-2-butenyl)-1H-isoindole-1,3(2H)-dione.

A mixture of 1.90g of (E)-2(3-formyl-2-butenyl)-1H-isoindole-1,3(2H)-dione (8.30 mmol), 0.52g (8.30 mmol) sodium cyanoborohydride and several drops of methyl orange in a 19:1 tetrahydrofuran-water mixture was stirred as 10 ml of a mixture of 1 ml of concentrated aqueous hydrochloric acid and 1 ml of glacial acetic acid in 8 ml tetrahydrofuran was added dropwise until the indicator turned red (pH=3.3). The mixture was then stirred for about three hours before being evaporated to dryness. The residue was taken up in 50 ml methylene chloride and 50 ml deionized water and the layers shaken together before being separated. The aqueous layer was washed with an additional 50 ml methylene chloride which was then combined with the organic layer. The organic solution was treated with magnesium sulfate and decolorizing charcoal and filtered before removing the solvent. The clear oil was allowed to stand and crystallized to form 1.99g white solid. The product was recrystallized from dichloromethane and hexane to yield 1.35g (E)-2(4-hydroxy-3-methyl-2-butenyl)-1H-isoindole-1,3(2H)-dione.

The resulting (E)-2(4-hydroxy-3-methyl-2-butenyl)-1H-isoindole-1,3(2H)-dione can be employed to produce trans-zeatin as previously described.

CLAIMS

1. A process for the production of (E)-2(4-hydroxy-3-methyl-2-butenyl)-1H-isoindole-1,3(2H)-dione comprising reacting a (E)-4-halo-2-methylbut-2-en-1-al or an aldehyde protected derivative thereof with an alkali metal phthalimide to form (E)-2(3-formyl-2-butenyl)-1H-isoindole-1,3(2H)-dione and reducing said product with a reducing agent capable of reducing the aldehyde moiety but not the imide moiety, with the proviso that when an aldehyde protective derivative of (E)-4-halo-2-methylbut-2-en-1-al is employed as the starting reactant the initial reaction product is hydrolyzed before being reduced.

2. The process of claim 1 wherein the starting reactants are (1) a compound of the formula

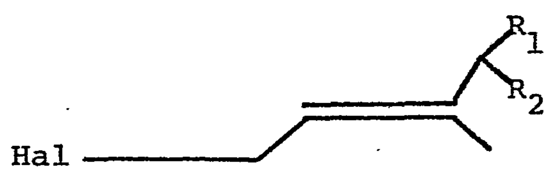

wherein Hal is chlorine or bromine and $R_1$ and $R_2$ are aldehyde protective groups or $R_1$ and $R_2$ taken together is =O and (2) a compound of the formula

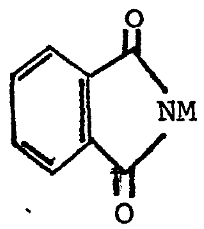

wherein M is an alkali metal.

3. The process of claim 2 wherein the reducing agent employed is sodium cyanoborohydride.

4. The process of claim 3 wherein the initial reactants are (E)-4-bromo-2-methylbut-2-en-1-al and potassium phthalimide.

5. The process of claim 3 wherein the initial reactants are (E)-4-bromo-1,1-diethoxy-2-methyl-2-butene and the initial reaction product is hydrolyzed to (E)-2(3-formyl-2-butenyl)-1H-isoindole-1,3(2H)-dione in an acetone-water mixture in the presence of p-toluene sulfonic acid.

European Patent Office

# EUROPEAN SEARCH REPORT

Application number

EP 83 10 1236

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. ³) |
| D,X | SYNTHESIS, no. 11, november 1972, pages 618-619 J. CORSE et al.: "An improved synthesis of trans-Zeatin" * Pages 618-619 * <br> ----- | 1-5 | C 07 D 209/48 |
| | | | TECHNICAL FIELDS SEARCHED (Int. Cl. ³) |
| | | | C 07 D 209/48 |

The present search report has been drawn up for all claims

| Place of search | Date of completion of the search | Examiner |
|---|---|---|
| THE HAGUE | 19-05-1983 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82